# EUROPEAN PATENT SPECIFICATION

(11) **EP 1 592 346 B1**
(45) Date of publication and mention of the grant of the patent: **20.07.2011**
(21) Application number: 03814778.1
(22) Date of filing: 15.12.2003
(51) Int. Cl.: A61B 5/15, A61M 5/32

(54) **SAFETY NEEDLE ASSEMBLY**
SICHERHEITSNADELANORDNUNG
ENSEMBLE AIGUILLE SECURISE

(30) Priority: 16.12.2002 US 433760 P
(43) Date of publication of application: 09.11.2005
(73) Proprietor: Becton Dickinson and Company, Franklin Lakes, New Jersey 07417-1880 (US)
(72) Inventor: CRAWFORD, Jamieson, W., M., New York, NY 10025 (US); BENNETT, Michael, Summit, NJ 07901 (US); NEWBY, C, Mark, Tuxedo, NY 19087 (US)
(74) Representative: von Kreisler Selting Werner
(86) International application number: PCT/US2003/039791
(87) International publication number: WO 2004/060161

(56) References cited:
- US-A- 5 171 303
- US-A- 5 188 611
- US-A- 5 665 075
- US-A- 5 885 249
- US-A1- 2002 151 853

## Description

### FIELD OF THE INVENTION

The present invention relates to a shield for a needle and more particularly to a safety shield assembly that may be used in conjunction with a syringe assembly, a hypodermic needle, a needle assembly, a needle assembly with a needle holder, a blood collection needle, a blood collection set, an intravenous infusion set or other fluid handing devices or assemblies that contain piercing elements.

### BACKGROUND OF THE INVENTION

Disposable medical devices having piercing elements for administering a medication or withdrawing a fluid, such as hypodermic needles, blood collecting needles, fluid handling needles and assemblies thereof, require safe and convenient handling. The piercing elements include, for example, pointed needle cannula or blunt ended cannula.

Safe and convenient handling of disposable medical devices is recognized by those in the medical arts so as to minimize exposure to blood borne pathogens. Safe and convenient handling of disposable medical devices results in the disposal of the medical devices intact.

As a result of this recognition, numerous devices have been developed for shielding needles after use. Many of these devices are somewhat complex and costly. In addition, many of these devices are cumbersome to use in performing procedures. Furthermore, some of the devices are so specific that they preclude use of the device in certain procedures or with certain devices and/or assemblies. For example, some devices employ very short thin needle cannulas. A shield designed to lock near the distal end of one needle cannula might not engage a much shorter needle cannula. Additionally, a shield designed to lock with a wider gange needles cannula might be more likely to generate a spray upon engaging a much narrower needle cannula. Furthermore, it may be desirable to reduce the force required to effect Shielding without reducing the audible and tactile indications of complete shielding.

Therefore, there exists a need for a safety shield assembly: (i) that is manufactured easily; (ii) that is applicable to many devices; (iii) that is simple to use with one hand; (iv) that can be disposed of safely; (v) that does not interfere with normal practices of needle use; (vi) that has tactile features whereby the user may be deterred from contacting the needle, the may easily orient the needle with the patient and easily actuate and engage the shield assembly; (vii) that has visual features Whereby the user may be deterred from contacting the needle, the user may easily orient the needle with the patient and easily actuate and engage the shield assembly; (viii) that is not bulky; (ix) that includes means for minimizing exposure to the user of residual fluid leaking from the needle; and (x) provides minimal exposure to the user because the needle shield is immediately initiated by the user after the needle is withdrawn from the patient's vein.

US 5,885,249, to which the preambles of claims 1 and 8 refer, discloses a syringe with a cap including a syringe main body having the needle attached to the extreme end thereof and a cap for covering the needle of the syringe main body, wherein the cap includes a cap securing portion secured to the extreme end of the syringe main body. The cap further includes the main body pivotally coupled with the cap securing portion through the hinge portion. The cap main portion includes an accommodating portion for accommodating the needle and a cut-out portion which communicates with the accommodating portion and into which the needle can go in and from which the needle can be taken out. The syringe with cap comprises securing elements capable of securing the cap to the cap securing portion when the syringe is used.

### SUMMARY OF THE INVENTION

The subject matter of the invention is defined by each of claims 1 and 8.

The present invention is a safety shield assembly that comprises: a shield; means for connecting the shield to a fluid handling device that contains a piercing element, such as needle; means for pivoting the shield away from the needle; means for securely covering and/or containing the needle within the shield and means for securely locking the shield in a final non-retractable closed position over the needle.

The shield preferably includes a plurality of cannula finger locks spaced axially along the shield for locked engagement with the cannula when the shield is in the final closed position around the needle cannula. Each cannula finger lock preferably projects obliquely from one sidewall of the shield angularly toward the opposed sidewall and the top wall of the shield. Each cannula finger lock has a base that projects rigidly from the respective sidewall and a flexible end that extends from the base. The rigidity of the base and the flexibility of the end may be achieved by relative dimensions along the cannula finger lock. Thus, the base of each cannula finger lock may be relatively thick and/or relatively wide. The end of each cannula finger lock may be tapered in at least one direction to be relatively thin and/or relatively narrow. The rigidity of the base is achieved by providing webs extending between the bases of adjacent cannula finger locks, but not extending between then ends of the cannula finger locks. The cannula finger locks are disposed and aligned such that the flexible ends contact the needle cannula when the shield approaches the final closed position. Contact between the cannula and the cannula finger locks will cause the flexible ends of the cannula finger locks to deflect resiliently toward the sidewall from which the cannula finger locks extend. Sufficient rotation of the shield will cause the needle cannula to pass the cannula finger locks. As a result, the flexible end of each cannula finger lock will resiliently return to or toward its undeflected condition for securely trapping the needle cannula in the shield. Forces in a direction to move the shield from the closed position to the open position will cause the needle cannula to engage the top face of each cannula finger lock. The oblique alignment of the cannula finger locks will cause the shield and/or the cannula to deflect slightly so that the cannula slides into engagement with the rigid base of the cannula finger lock. This distinguishes from structures where the entire cannula finger lock is deflectable about its base and/or where the entire cannula finger lock is deflectable at any location along its length.

Preferably, the shield comprises a rearward end, a forward end, a slot or longitudinal opening for housing the used needle in the forward end, means for securing the needle in the slot, means for guiding the needle into the slot, means for connecting the shield and the fluid handling device, means for guiding the user's fingers to move the shield into various positions, and means for retaining the shield securely over the used needle.

Desirably, the means for connecting the shield to the fluid handling device is a collar. Preferably, the shield is connected movably to a collar which is connected to a fluid handling device.

Preferably, the shield is connected to the collar by a hanger bar that engages with a hook arm on the collar so that the shield may be pivoted with respect to the collar into several positions. It is within the purview of the present invention to include any structure for connecting the shield to the collar so that the shield may be pivoted with respect to the collar. These structures include known mechanical hinges and various linkages, living hinges, or combinations of hinges and linkages.

Most preferably, the shield is connected to the collar by an interference fit between the hanger bar and the hook bar. Therefore, the shield always is oriented in a stable position and will not move forward or backwards unless movement of the shield relative to the hanger bar and the hook bar is initiated by the user.

Alternatively, the shield and collar may be a unitary one-piece structure. The one-piece structure may be obtained by many methods, including molding the shield and the collar as a one-piece unit, thereby eliminating the separate shield and collar during the manufacturing assembly process.

The assembly of the present invention may further comprise tactile and visual means for deterring the user from contacting the needle, providing easy orientation of the needle with the patient and providing the user with a guide for actuation and engagement with the shield.

The assembly of the present invention may further comprise means for minimizing exposure by the user to residual fluid leaking from a used needle. For example, a polymer material, such as a gel, may be located in the shield.

Most desirably, the assembly of the present invention is such that the cooperating parts of the assembly provide the means for the shield to move into a forward position over the needle. Thus, by simple movement of the shield into a forward position over the used needle, the assembly is ready for subsequent disposal.

Therefore, the safety shield assembly of the present invention provides minimal exposure of the user to a needle because the shielding is initiated by the user immediately after the needle is withdrawn from the patient's vein.

Desirably, the assembly of the present invention may be used with a syringe assembly, a hypodermic needle, a needle assembly, a needle assembly with a needle holder, a blood collection set, an intravenous infusion set or other fluid handling devices. Preferably, the assembly of the present invention is used with a needle assembly comprising a needle and a hub. Preferably the needle is a conventional double ended needle.

Most preferably, the present invention is used with a needle assembly comprising a hub and a needle connected to the hub whereby the needle comprises a non-patient end and an intravenous end. The collar of the present invention may comprise a hook arm and the shield may be connected movably to the hook arm. Thus the shield may be pivoted with respect to the collar and moved easily into several positions.

Preferably, the collar is fitted non-rotatably with the hub of the needle assembly. Additionally, the collar includes cooperating means that mate with reciprocal means on the shield to help retain the shield in a final closed position, to propel the shield toward the final closed portion and to provide a clear audible and tactile indication of complete shielding. The cooperating means on the collar may include generally chevron-shaped projection formed on a side of the collar substantially diametrically opposite the hook arm or other such structure that provides the hinge connection to the shield. The chevron-shaped structure includes a forward or distal point. Slanting surfaces diverge and extend proximally from the distal point. The slanting surfaces cooperate with the reciprocal means on the shield to generate a deflection of the sidewalls of the shield away from one another. The chevron-shaped structure further includes proximal ends that are convexly arcuate. The convexly arcuate ends of the chevron-shaped structure on the collar cooperate with the reciprocal means on the shield and with the resiliently deflectable sidewalls of the shield to propel the shield toward its closed position, to assist in holding the shield in its closed position and to generate the tactile and audible indication of shielding.

Preferably, the collar is fitted with the hub of the needle assembly so that the collar cannot rotate around the hub. Additionally, the collar includes cooperating means that mate with reciprocal means on the shield to propel the shield toward a final closed position.

Alternatively, the collar and hub may be a unitary one-piece structure. The one piece structure may be accomplished by many methods including molding the collar and the hub as a one-piece unit thereby eliminating the need to separately assemble the collar to the hub during the manufacturing process.

Most preferably, the collar is fitted with the hub of the needle assembly so that the bevel surface or bevel up surface of the intravenous or distal end of the needle faces the same side of the collar when the shield is in the open position. Alignment of the collar, hub, shield and needle with the bevel surface up makes it easier to insert the needle into the patient without manipulating the assembly. The orientation of the intravenous end of the needle with the bevel up assures the user that the needle is properly oriented properly for use and does not require any manipulation before use. Most notably, the orientation of the shield provides a visual indication to the user of the orientation of the bevel surface of the needle.

Preferably, the shield is capable of pivoting from an open position where the intravenous end of the needle is exposed and bevel up, to an intermediate position where the needle is partially covered, to a final closed nonretractable position where the needle is covered completely and the shield is locked and no longer able to be moved out of the closed position.

Alternatively, it is within the purview of the present invention that the shield, collar and hub is a unitary one-piece structure. The one-piece structure may be accomplished by many methods including molding the shield, collar and hub as a one-piece unit thereby eliminating the need to separately assemble the shield, collar and hub during the manufacturing process.

It is an advantage of the present invention that the shield covering the used intravenous end of the needle provides easy containment of the used needle. A further advantage of the shield is that it will only move upon initiation by the user.

The assembly of the present invention when used with a fluid handling device is also easily disposable when removed from a conventional needle holder, or other such device.

Another important feature of the present invention includes means for locking the shield in a closed permanent position covering the needle. The closed permanent position will generally withstand the normal forces encountered during proper disposal of the safety shield assembly when it is removed from a conventional needle holder.

A notable attribute of the present invention is that it is easily adaptable with many devices. For example, the invention is usable with syringe assemblies, hypodermic needles, needle holders, blood collection needles, blood collection sets, intravenous infusion sets such as catheters or other fluid handling devices or assemblies that contain piercing elements.

Another notable attribute of the present invention is that the tactile and visual features deter the user from touching the needle, allow the user to easily orient the needle with the patient and guide the user to actuate and engage the shield of the assembly.

### BRIEF DESCRIPTION OF THE DRAWINGS

FIG. 1 is a perspective view of the safety shield assembly of the present invention as connected to a needle assembly and related packaging features.
FIG. 2 is a perspective view of the unassembled pieces of FIG. 1.
FIG. 3A and 3B are bottom views of the shield as shown in FIG. 2.
FIG. 4 is a cross sectional view of the collar as shown in of FIG. 2 taken along lines 4-4 thereof.
FIG. 5 is a cross sectional view of the needle hub as shown in FIG. 2 taken along lines 5-5 thereof.
FIG. 6 is a cross sectional view of the shield of FIG. 2 taken along lines 6-6 thereof.
FIGS. 7-12 illustrate the use of the safety shield assembly with the needle assembly of FIG. 1 with a conventional needle holder.
FIG. 13 is a cross sectional view of the assemblies in use with a conventional needle holder as shown in FIG. 12 taken along lines 13-13 thereof.
FIGS. 14A and 14B are cross-sectional views of the assembly of FIG. 13 taken along lines 14-14 thereof at rotational positions immediately prior to full shielding and immediately after full shielding.
FIGS. 15A and 15B are bottom views of the assembly as shown in FIG. 11.
FIG. 16 illustrates an additional embodiment of the present invention, whereby a gel material is located in the shield as shown in a bottom view of the assemblies of FIG. 11.
FIG. 17 is a perspective view of an additional embodiment of the present invention in use with a blood collection set.
FIG. 18 is a perspective view of an additional embodiment of the present invention in use with a syringe.
FIG. 19 is a perspective view of an additional embodiment of the present invention in use with a catheter.

### DETAILED DESCRIPTION OF THE INVENTION

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope of the invention. The scope of the invention will be measured by the appended claims and their equivalents.

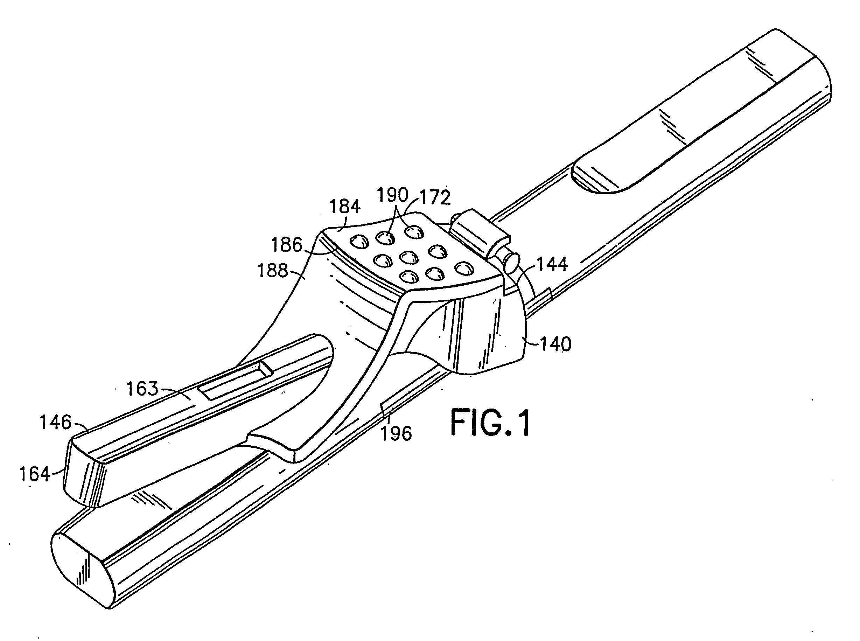

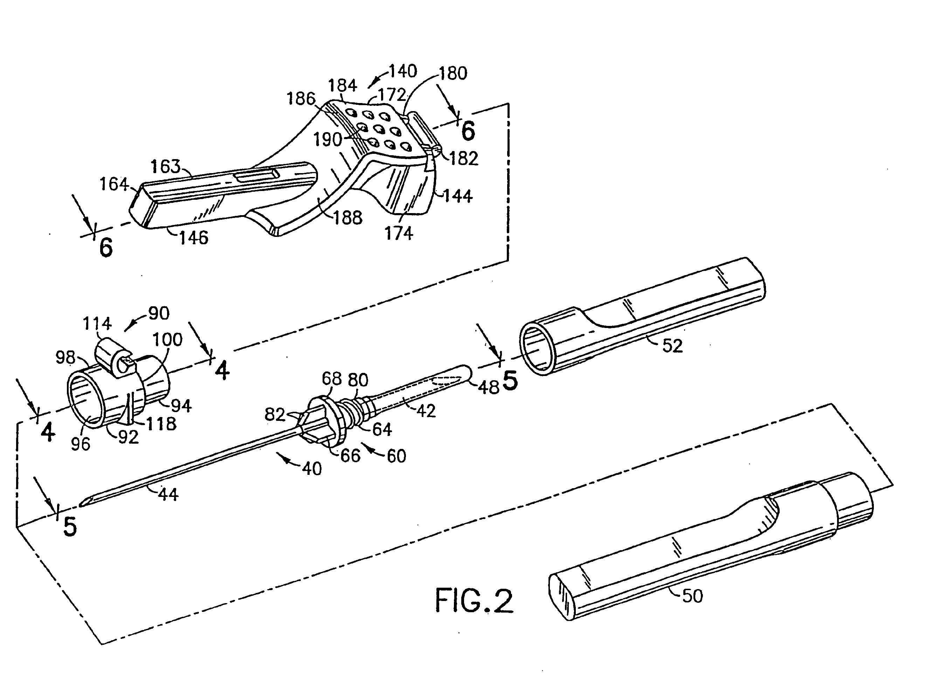

Referring to the drawings in which like reference characters refer to like parts throughout the several views thereof, FIGS. 1 and 2 illustrate a needle assembly with the safety shield assembly of the present invention and the related packaging features. The needle assembly includes a needle **40,** a hub **60,** packaging features to cover the needle and a label. The safety shield assembly includes a collar **90** and a shield **140.**

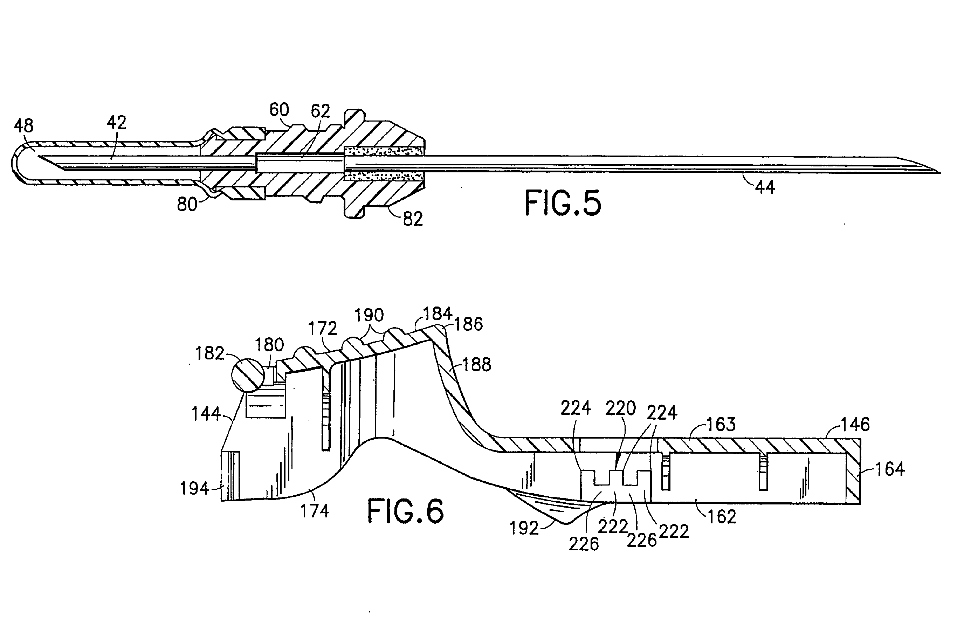

As shown in FIG. 2 and 5, needle 40 includes a non-patient end **42,** an intravenous end **44** and a passageway **46** extending between the non-patient end and the intravenous end. An elastomeric sleeve **48** covers the non-patient end. A first rigid sleeve **50** covers the intravenous end and a second rigid sleeve **52** covers the non-patient end and the elastomeric sleeve. As shown in FIG. 1, a label **196** may also be applied to the finally assembled parts.

As shown in FIGS. 2 and 5, hub **60** includes a threaded end **64,** a ribbed end **66** and passageway **62** extending between the threaded end and the ribbed end. Threaded end **64** and ribbed end **66** are separated by flange **68.** Non-patient end **42** of needle **40** extends from threaded end **64** and intravenous end **44** of needle **40** extends from ribbed end **66.** Preferably, threaded end **64** comprises male threads **80** for mounting the hub on a conventional needle holder and ribbed end **66** comprises male ribs **82** for connecting the hub and collar **90.**

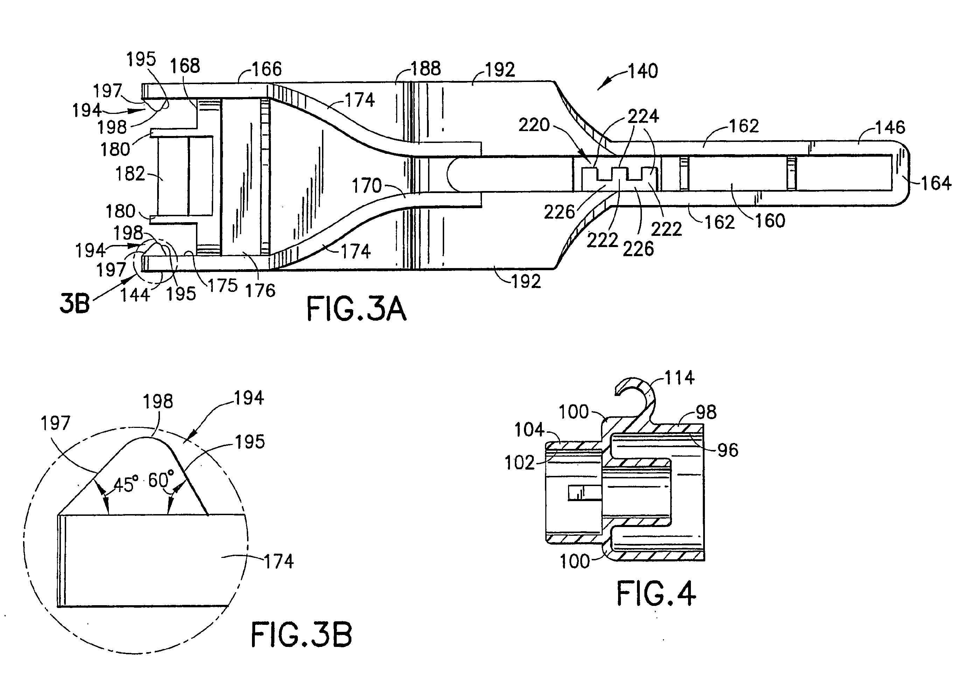

As shown in FIGS. 2 and 4, collar **90** includes a forward skirt **92** and a rearward skirt **94.** Forward skirt **92** is cylindrical and comprises an inner circumferential surface **96** and an outer circumferential surface **98.** Forward shirt **92** mates with rearward skirt **94** at a shoulder **100.** Rearward skirt **94** is cylindrical and comprises an inner circumferential surface **102** and an outer circumferential surface **104** and extends from shoulder **100** opposite of forward skirt **92.** The inner diameter of forward skirt **92** is larger than the inner diameter of rearward skirt **94.** Alternatively, the inner diameters for collar **90** can be equal. A hook **114** extends from outer circumferential surface **98** of forward skirt **92.** Additionally a chevron-shaped protrusion **118** projects outwardly from outer circumferential surface **98** of forward skirt **92** at a side opposite hook **114.** The chevron-shape protrusion **118** is substantially symmetrical and has an peak **120** pointed toward forward skirt **92** and ramp surfaces **122** that diverge symmetrically from peak **120** toward rearward skirt **94.** Ramp surfaces **122** terminate at rounded ends **124** at the outer side and proximal extremes of chevron-shaped protrusion **118.** Rounded ends **124** extend continuously into the proximal side of chevron-shaped protrusion **118** facing toward rearward skirt **94.**

As shown in FIGS. 2 and 6, shield **140** comprises a rearward end **144** and a forward end **146.**

Forward end **146** of shield **140** includes a slot or longitudinal opening **160** formed by sidewalls **162** that extend downwardly from top wall **163** and run substantially opposite of one another in parallel along the length of slot **160** towards forward end wall **164.** Slot **160** is slightly wider than needle **40.** Sidewalls **162** include bottom edges **165** that extend substantially parallel to one another and parallel to top wall **163.**

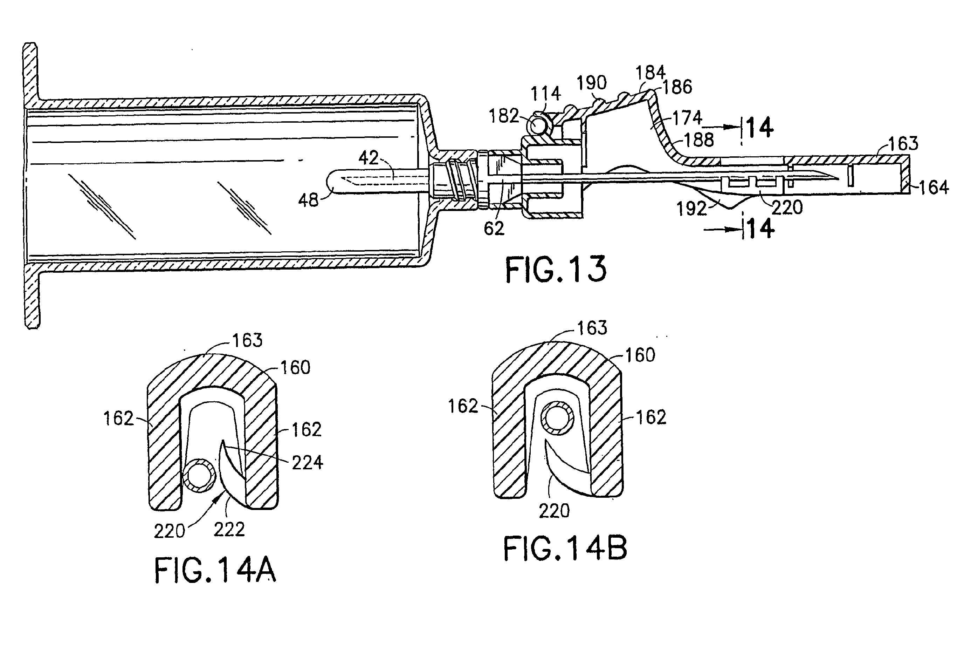

Three cannula finger locks **220** are located at axially spaced locations along one of sidewalls **162** and are configured to secure the used needle. Each cannula finger lock **220** extends from a location on a first of the sidewalls **162** adjacent the bottom edge **165** thereof and projects angularly toward the opposed sidewall **162** and toward the top wall **163,** as shown in FIGS. 14A and **14B****.** The projection of each cannula finger lock **220** from the respective sidewall **162** preferably exceeds half the distance between the respective sidewalls. Each cannula finger lock **220** includes a base **222** that projects unitarily and rigidly from portions of the respective sidewall **167** adjacent bottom edge **165** thereof. Each cannula finger lock **220** further includes an end **224** that extends unitarily and flexibly from base **222** of the respective cannula finger lock. As shown most clearly in FIGS. 14A and 14B, bases **222** are substantially thicker than ends **224** as measured in directions substantially perpendicular to the planes of cannula finger locks **220.** Additionally, bases **222** of cannula finger locks are unitarily joined to one another by webs **226.** The greater thickness dimensions of bases **222** and the existence of webs **226** substantially prevents bases **222** from flexing in either direction in response to opposed forces between cannula **40** and cannula finger locks **220** during movement of shield **140** in either an opening or closing direction. However, ends **224** of cannula finger locks 220 are deflectable relative to bases **226** as the needle enters slot **160** of shield **140.** Once the needle passes the ends of cannula finger locks **220,** flexible ends **224** of the cannula finger locks move back to their original positions relative to rigid bases **222** so that the needle is permanently trapped in slot **160** by cannula finger locks **167.**

Rearward end **144** of shield **140** defines a collar engaging area **166** that is a continuation of slot **160.** Collar engaging area **166** includes a rearward end **168,** a forward end **170,** a top finger guide area **172,** sidewalls **174** that extend downwardly from top finger guide area **172,** an underside area **176** dimensioned for surrounding collar **90,** and extending arms **180** to support hold hanger bar **182.** Sidewalls **174** are spaced apart by a major width adjacent rearward end **168.** The major width is selected to enable sidewalls **174** to slide across diametrically opposite side surfaces of forward skirt **92** of collar **90.** Sidewalls **174** converge, however, toward forward end **170** to define a minor distance therebetween substantially equal to the distance between sidewalls **162** at forward end **146** of shield **140.** Sidewalls **174** include bottom edges **177** that face away from top finger guide area **172.** As shown most clearly in FIG. 6, bottom edges **177** curve toward top finger guide area **172** at locations between rearward end **168** and forward end **170** of collar engaging area **166.**

The extreme rear ends of sidewalls **174** on collar engaging area **166** include rounded ears **194** that project toward one another from opposed inner surfaces **175** of sidewalls **174.** Rounded ears **194** are disposed to engage chevron-shaped projection **118** on collar 90. More particularly, each rounded ear **194** includes a distal surface **195,** a proximal surface **197** and a curved surface **198** extending between distal and proximal surfaces **195** and **197.** Distal surface **194** is aligned to sidewall **174** at a rake angle of approximately 60° and proximal surface **197** is aligned to sidewall **174** at an angle of approximately 45°. Curved surface **198** extends smoothly and convexly between distal and proximal surfaces **195** and 197. Proximal surfaces **197** of rounded ears **194** will engage ramp surfaces **122** of chevron-shaped projection **118** to deflect sidewalls **174** slightly away from one another as shield **140** approaches the second position. This deflection of sidewalls **174** will occur substantially simultaneously with the deflection of cannula finger locks **220.** The apex of curved surface **198** on each rounded ear **194** passes the respective rounded end surface **124** on chevron-shaped projection **118** on collar **90** slightly before cannula finger locks **220** pass the needle cannula. As a result, sidewalls **174** begin to return resiliently toward an undeflected condition. This resilient return of sidewalls **174** cooperates with raked distal surfaces **195** on rounded ears **194** to propel shield **140** into the second position where cannula finger locks **220** pass needle **40.** This acceleration of shield **140** caused by the resilient return of sidewalls **174** and raked distal surface **195** of ears **194** also causes sidewalls **174** to snap against chevron-shaped projection **118.** This snapping action provides a clear audible and tactile indication of complete shielding and occurs substantially when the used needle is trapped by cannula finger locks **220.** The angles of distal and proximal surfaces **195** and **197** of rounded ears **194** affects the performance of shield **140.** In particular, a smaller acute angle alignment of proximal face **197** reduces the force required to move shield **140** passed rounded ears **194.** A larger acute angle proximal surface **197** of rounded ears **194** requires a greater force to move shield **140** toward the second position. Similarly, the angle between distal surface **195** and sidewall **174** affects the acceleration characteristics as shield **140** is propelled toward the second position in response to the resilient return of sidewalls **174.**

Top finger guide area **172** comprises a first ramp **184** that extends slightly on an upwardly slope from the rearward end of the collar engaging area to a shoulder **186.** From shoulder **186** extends a second ramp **188** which slopes downwardly towards top section **163.** Most preferably, first ramp **184** comprises touch bumps **190.** The touch bumps provide a tactile and visual guide to alert the user that the user's finger has contacted the shield and that the shield is in a defined or controlled position. The touch bumps may be any configuration so long as they extend and are distinct from the top finger guide area. The touch bumps may also be of a distinguishing color as compared to the top finger guide area or the shield.

Second ramp **188** has interior surface **192** for urging the needle toward the center of slot **160** as the shield is being rotated into the closed position. The exterior surfaces are slightly inclined and extending radially from the second ramp. The interior surfaces are especially helpful if the longitudinal axis of the needle is misaligned with respect to the longitudinal axis of the hub.

Extending arms **180** are located at rearward end **168** and at the beginning of top finger area **172** and hold hanger bar **182.**

The safety shield assembly and the needle assembly are assembled together whereby needle **40** is connected to hub **60** and sealed with adhesive at the ends of the hub. Hub **60** is then joined with collar **90** by ultra-sonic welding techniques or any other bonding techniques, or mechanical fit, whereby rearward annular skirt **94** of collar **90** mates with ribbed end **66** of the hub. Male ribs **82** of the hub are contained or forced fitted within inner sidewall **102** of rearward annular skirt **94** of collar **90.** The collar is aligned with the intravenous end of the needle whereby the hook arm is aligned with the bevel up of the needle. Then rigid sleeve 50 is force fitted into inner side wall 96 of forward skirt **92** of collar **90** to cover the needle. Thereafter, shield **140** is connected to collar **90** whereby hanger bar **182** is force fitted into hook member **114** whereby slot **160** faces rigid sleeve **50.** Most preferably, the shield is connected to the collar by a force fit or interface fit between the hanger bar and the hook bar. Therefore, the shield is always oriented in a stable position and will not move unless movement of the shield is positively initiated by the user. To assemble the last piece, shield **140** is moved towards rigid sleeve **50** and second rigid sleeve **52** is force fitted onto outer sidewall **104** of rearward skirt **94** of collar **90.**

In addition, a label **196** may be applied to the finally assembled parts. The label may be used to prevent tamper resistance of the parts, so that they are not reused.

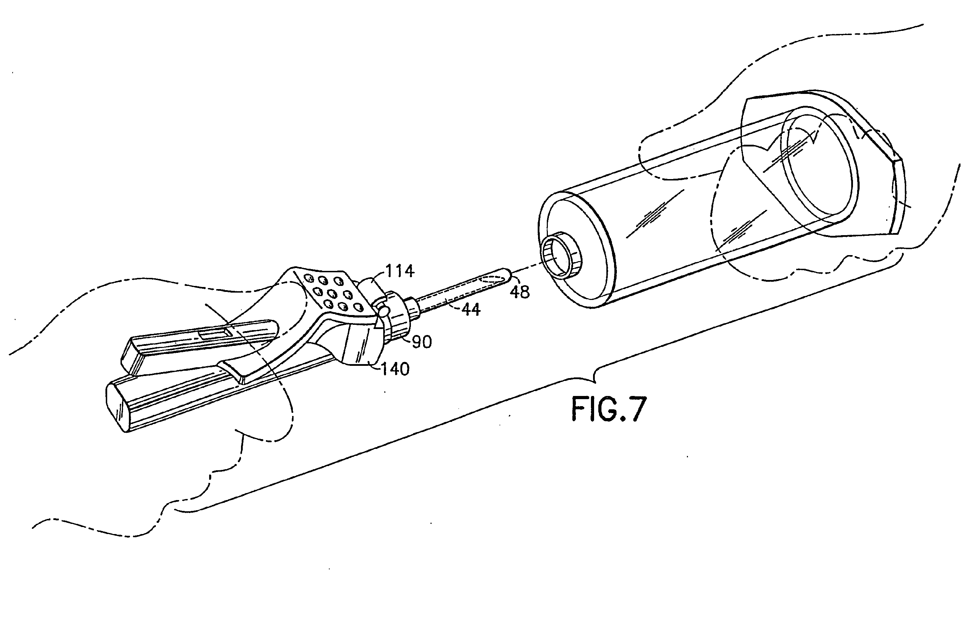

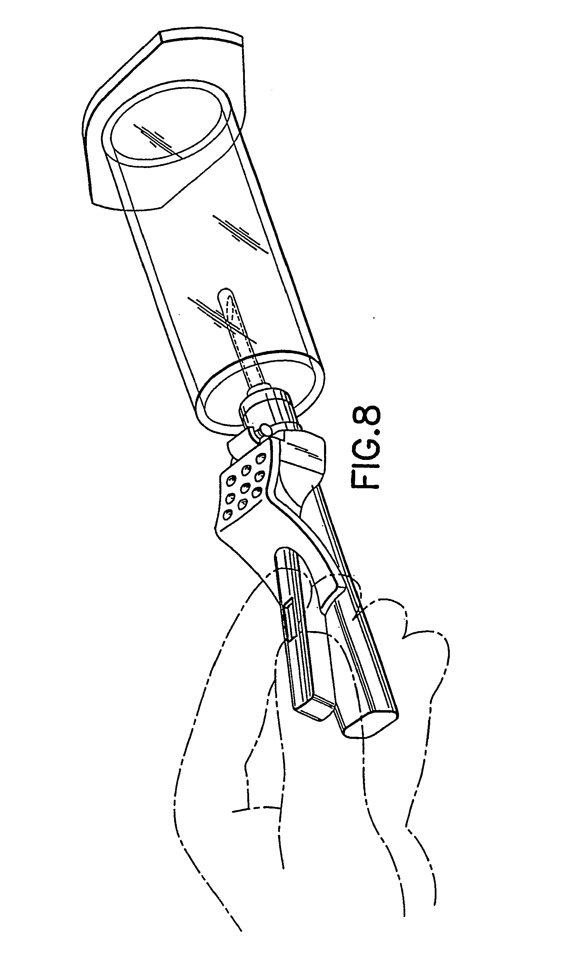

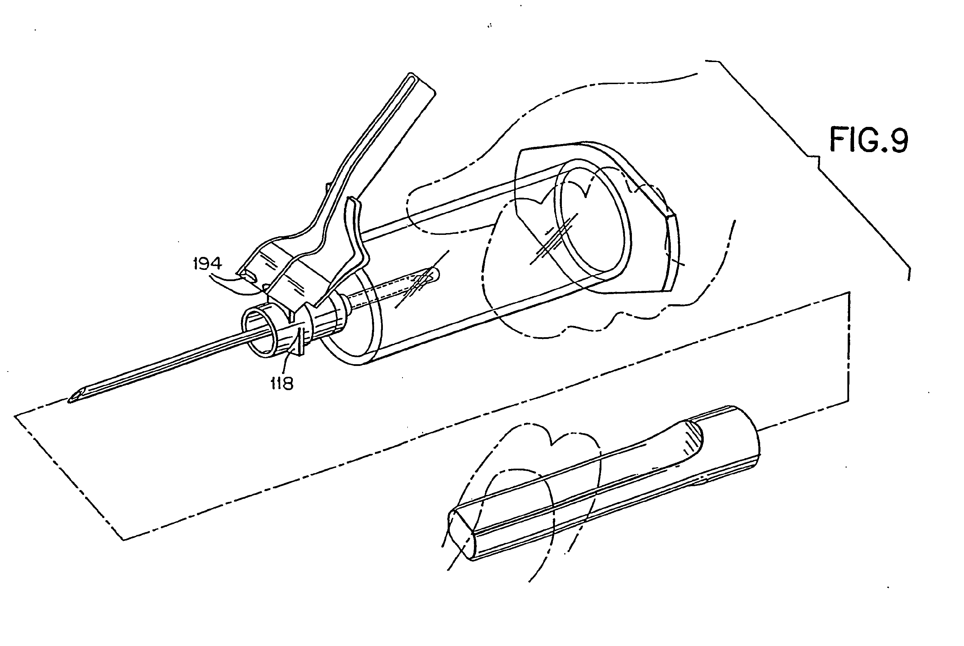

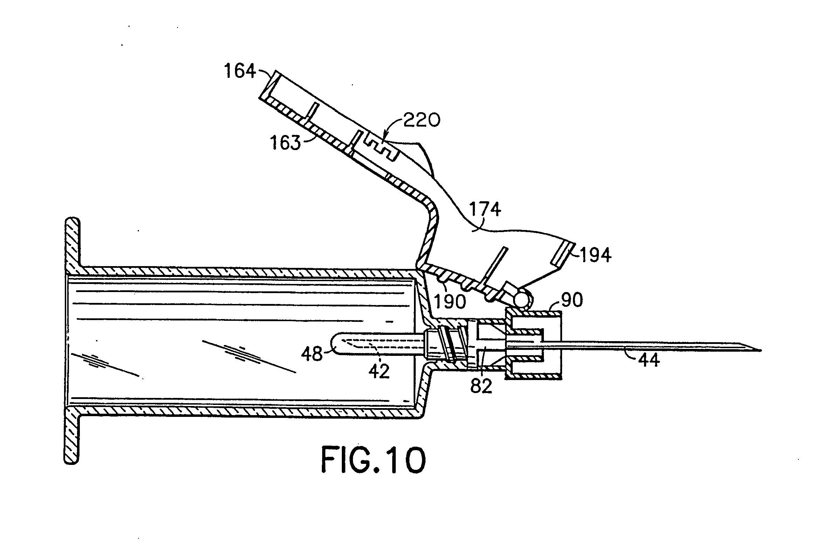

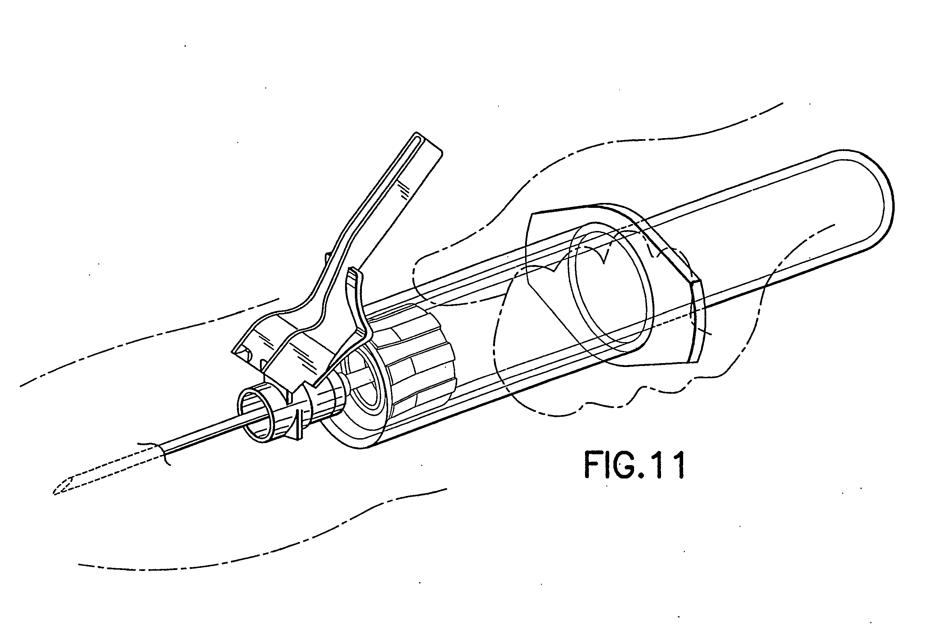

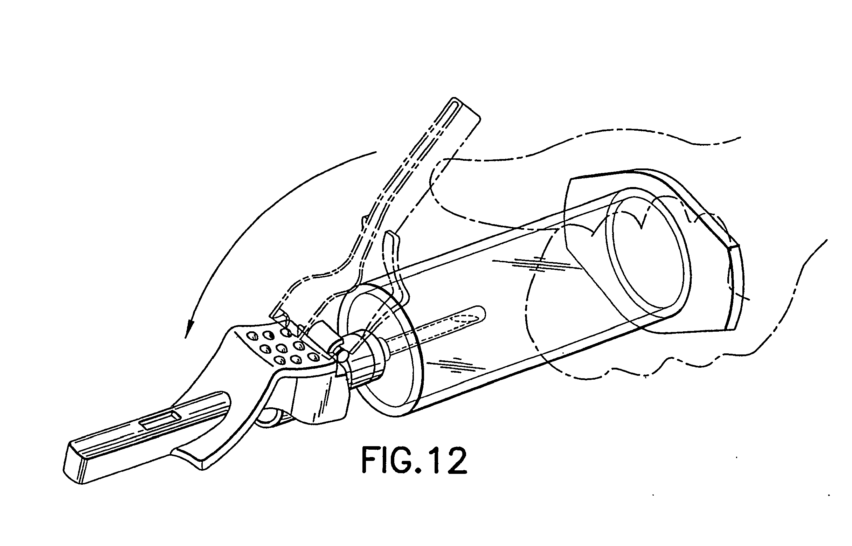

In use, as shown in FIGS. 7-15, the non-patient needle shield is removed and then a needle holder is screwed onto the hub of the needle. As specifically shown in FIGS. 8 and 12 the shield is then rotated back by the user towards the needle holder. Then as shown in FIG. 9, the intravenous needle shield is removed from covering the intravenous needle. Then as shown in FIG. 10, a venipuncture is conducted whereby the intravenous end of the needle is inserted into a vein of a patient and an evacuated tube having a closure is inserted into the needle holder. Then as shown in FIGS. 11 and 13, when the venipuncture is complete the user easily rotates the shield from the open position towards the intravenous needle to an intermediate position and then the user pushes on the shield at the top finger guide area to move the shield into a final, non-retractable locked position whereby the needle is trapped in the longitudinal opening. More particularly, needle **40** contacts flexible ends **224** of cannula finger locks **220.** The engagement of needle **40** with cannula finger locks **220** causes flexible ends **224** of cannula finger locks **220** to deflect relative to rigid bases **222** toward top wall **163** and toward sidewall **162** from which cannula finger locks **220** project. Sufficient rotation of shield **140** will cause needle **40** to pass cannula finger locks **220.** As a result, flexible ends **224** of all three cannula finger locks **220** will return resiliently to an undeflected condition relative to rigid bases **222.** Thus, needle **40** will be trapped above cannula finger locks **220.** The angular alignment and relative dimensions of each cannula finger lock **220** provides good resistance to forces that tend to move shield **140** back toward the open position. More particularly, forces in an opening direction of shield **140** will cause engagement between needle **40** and the upper surface of flexible ends **224** of cannula finger locks **220.** The angular alignment of cannula finger locks **220** will cause slight deflection of needle **40** and shield **140** that will cause relative sliding movement between needle **40** and cannula finger locks **220.** As a result, needle **40** will slide into contact with rigid base **222** and web **226.** Hence, shield **140** cannot be returned to the opened condition.

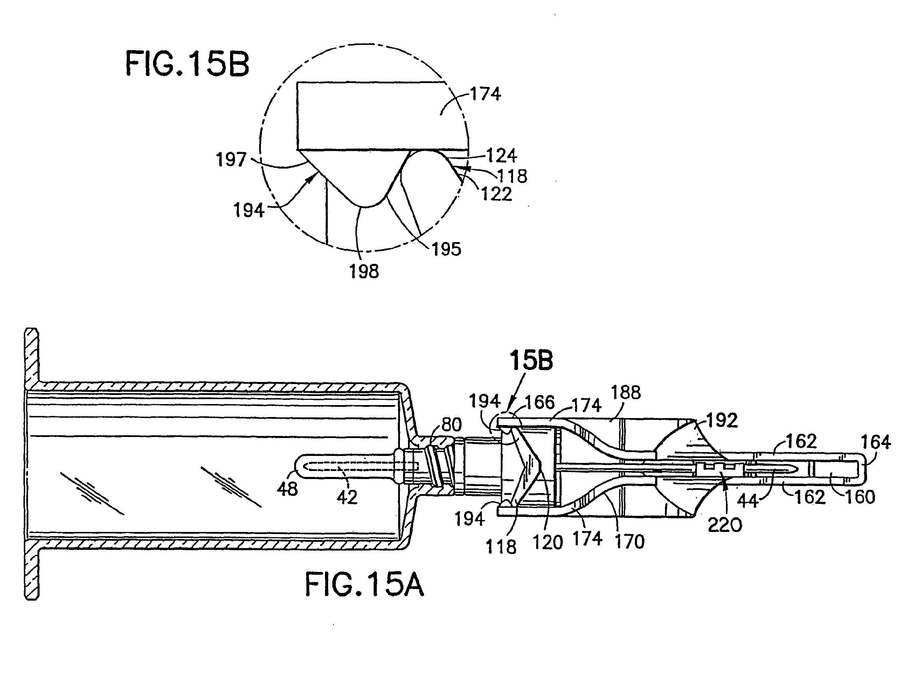

Needle **40** is contained within shield **140** as the shield is pivoted into the closed position. More particularly, proximal surfaces **197** of rounded ears **194** move over ramp surfaces **122** of chevron-shaped projection **118** and cause sidewalls 174 to deflect away from one another. The angularly aligned proximal faces **197** of rounded ears **194** ensures easy movement of shield **140.** Additionally, the resiliency of sidewalls **174** and the angular alignment of distal surface **195** of ears **194** causes a cooperation with rounded proximal ends **124** of chevron-shaped projection **118** to accelerate shield **140** into the full shielding closed position. Thus needle **40** snaps past cannula finger locks **220** and is trapped as shown in FIGS. 14 and 15 to ensure complete locking of shield **140** in the closed position. This accelerated movement of shield **140** helps to generate a clear audible and tactile indication of complete shielding.

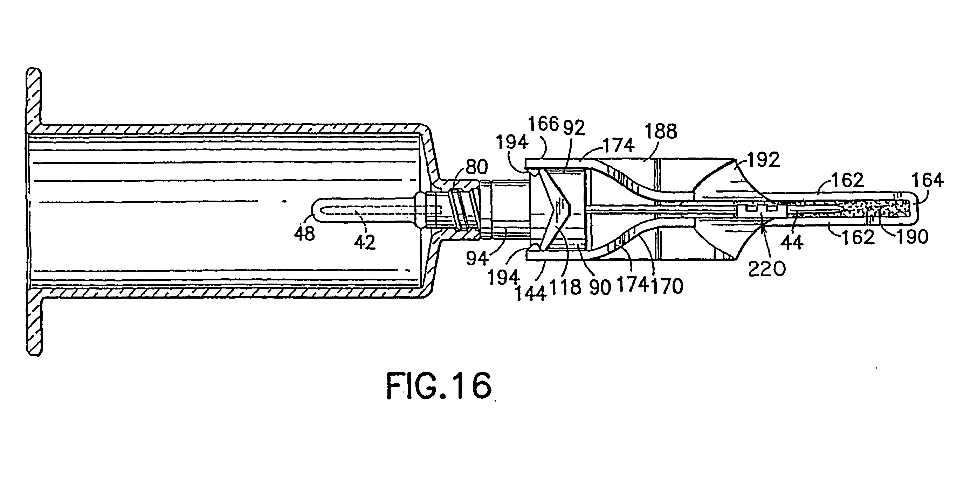

Alternatively as shown in FIG. 16, a gel material **190** is located in shield **140** so that when the needle snaps past cannula finger locks **220** it will come to rest in gel material **190.** The gel material will contain any residual fluid that may be on the needle. Simultaneously, rounded ears or projections **198** move over rounded proximal ends **124** of chevron-shaped projection **118.** This causes sidewalls **174** to deflect away from one another and then to snap back into engagement with collar **90** to provide a clear audible and tactile indication of complete shielding.

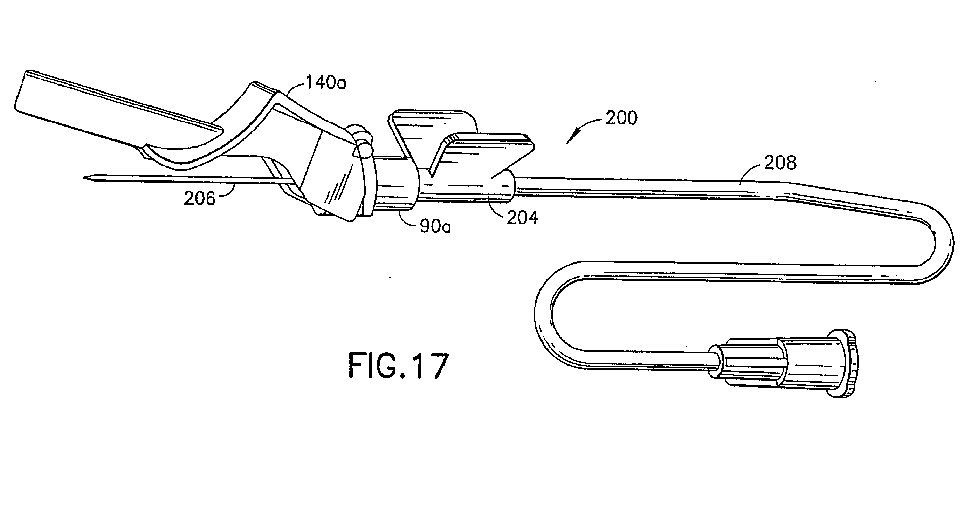

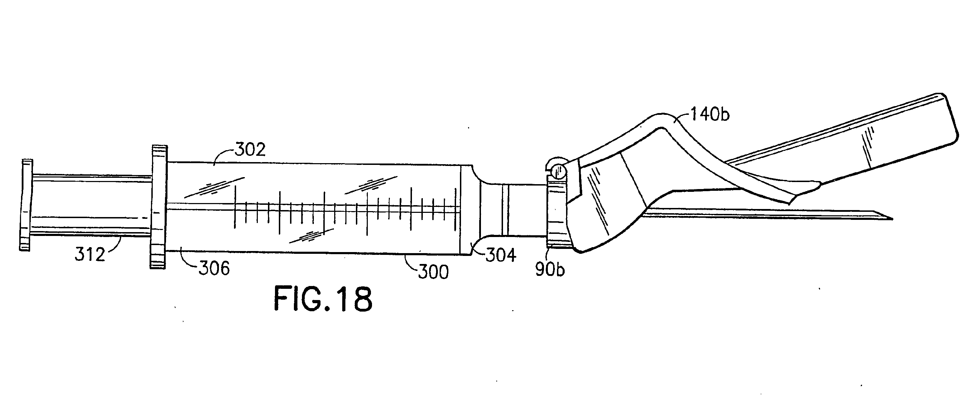

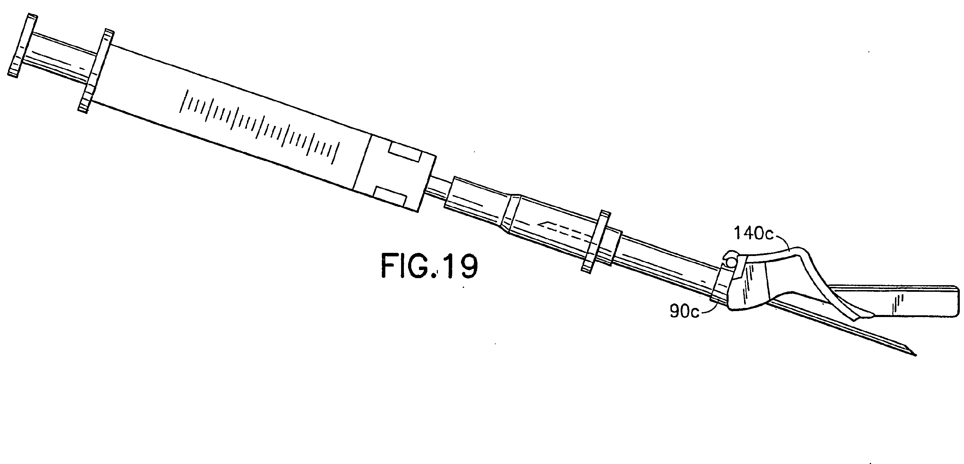

FIGS. 17, 18, and 19 are further embodiments of the invention that include may components which are substantially identical to the components. FIGS. 1-3. Accordingly, similar components performing similar functions will be numbered identically to those components of FIGS. 1-3, except that a suffix "a" will be used to identify those similar components in FIG. 17, a suffix "b" will be used to identify those similar components in FIG. 18 and a suffix "c" will be used to identify those similar components in FIG. 19.

Alternatively, the safety shield assembly of the present invention may be used in conjunction with a conventional intravenous (IV) fusion set, as illustrated in FIG. 17.

For purposes of illustration, shield **140a** and collar **90a** are connected to a conventional IV infusion set, **200,** or butterfly structure comprising a needle body with a needle hub **204** extending from the forward end of the needle body and a needle 206 embedded in hub **204.** Extending from the rearward end of the needle body is flexible tubing **208** which is conventional and utilized to allow the user to manipulate the structure and to connect it subsequently to supplies of infusion liquids or for the return of collected blood if the arrangement is being used to collect blood.

Infusion set **200** further comprises flexible wings **210** attached to and projecting outwardly from needle hub **204.**

Alternatively, the safety shield assembly of the present invention may be used in conjunction with a syringe, as illustrated in FIG. 18.

For purposes of illustration, shield **140b** and collar 90b are connected to a conventional hypodermic syringe **300** comprising a syringe barrel **302** having a distal end **304** a proximal end **306** and a plunger **312.**

Alternatively, the present invention may be used in conjunction with a catheter as illustrated in FIG. 19.

The shield and collar of the safety shield assembly of the present invention are comprised of moldable parts which can be mass produced from a variety of materials including, for example, polyethylene, polyvinyl chloride, polystyrene or polyethylene and the like. Materials will be selected which will provide the proper covering and support for the structure of the invention in its use, but which will provide also a degree of resiliency for the purpose of providing the cooperative movement relative to the shield and the collar of the assembly.

## Claims

1. A safety needle assembly comprising a needle hub (60) with proximal and distal ends (64,66) and a passage (62) extending between said ends, a needle cannula (40) mounted to said passage (62) of said needle hub (60) and having a pointed distal end (44) projecting beyond said distal end (66) of said hub (60), a shield (140) having proximal and distal ends (144,146), said proximal end (144) of said shield (140) being hingedly mounted to said hub (60) for rotation from a first position where said shield is spaced from said needle cannula to a second position where said shield substantially surrounds said needle cannula, said shield (140) comprising a top wall (163) and opposed first and second sidewalls (162) extending from said top wall (163), said sidewalls (162) having edges (165) remote from said top wall (163), at least one cannula finger lock (220) having a base end (222) integral with the first sidewall (162) and a free end (224) projecting angularly towards said top wall (163), portions of said cannula finger lock (220) in proximity to said base end (222) thereof being substantially rigid, and portions of said cannula finger lock (220) in proximity to said free end (224) thereof being resiliently deflectable, whereby resiliently deflectable portions of said cannula finger lock (220) yield as said shield (140) is rotated into said second position to permit said needle cannula (40) to be trapped between said cannula finger lock (220) and said top wall (163), and whereby substantially rigid portions of said cannula finger lock (220) prevent re-exposure of said needle cannula (40) after said shield (140) is in said second position,
**characterized in that**
said at least one cannula finger lock (220) comprises at least first and second cannula finger locks,
a web (226) extends between portions of said first and second cannula finger locks (220) substantially adjacent the respective base ends (222) thereof for making said cannula finger locks (220) substantially rigid at locations in proximity to said base ends (222) thereof, and
portions of said cannula finger locks (220) adjacent the free ends (224) thereof are substantially free of said webs (226).

2. The safety needle assembly of claim 1, wherein said portions of said cannula finger lock (220) in proximity to said free end (224) are made more flexible than portions of said cannula finger lock (220) near said base end (222) by forming said cannula finger lock with reduced dimensions at locations in proximity to said free end (224) thereof.

3. The safety needle assembly of claim 2, wherein portions of said cannula finger lock (220) in proximity to said free end (224) thereof are resiliently deflectable in directions transverse to said needle cannula (40) when said shield (140) is rotated into said second position, said portions of said needle cannula (40) in proximity to said free end (224) being of reduced dimension in directions extending substantially along a direction of deformation of said cannula finger lock (220).

4. The safety needle assembly of claim 2, wherein portions of said cannula finger lock (220) in proximity to said free end (224) thereof are resiliently deflectable in directions transverse to said needle cannula (40) when said shield (140) is rotated into said second position, said portions of said needle cannula (40) in proximity to said free end (224) being of reduced dimension in directions substantially parallel to said needle cannula (40) when said shield (140) is in said second position.

5. The safety needle assembly of claim 1, wherein said cannula finger lock (220) is formed unitarily with said shield (140).

6. The safety needle assembly of claim 1, wherein said needle cannula (40) has a proximal end (42) projecting proximally beyond said hub (60).

7. The safety needle assembly of claim 1, wherein said hub (60) further includes at least one detent (118) formed thereon, and wherein said shield (140) includes an ear (194) formed thereon and configured for engaging said detent on said hub (60) when said shield (140) is in said second position, said detent (118) and said ear (194) being configured for providing audible and tactile indication of said shield (140) reaching said second position.

8. A safety needle assembly comprising a medical device (200,300), a needle cannula (40,206) mounted to the medical device (200,300) and having a pointed distal end (44) projecting beyond said medical device, a shield (140a,140b) having an elongate slot (160), said slot (160) being dimensioned for receiving at least said pointed distal end (44) of said needle cannula (40,206), said shield (140a,140b) being hingedly mounted in proximity to said medical device (200,300) for rotation from a first position where said needle cannula is exposed to a second position where at least said pointed distal end (44) of said needle cannula is in said slot (160) of said shield (140), at least one cannula finger lock (220) having a substantially rigid base end (222) projecting from said shield (140a,140b) and partly into said slot (160), said cannula finger lock (220) further having a resiliently deflectable free end (224), whereby said resiliently deflectable free end (224) of said cannula finger lock (220) yields as said shield (140a,140b) is rotated into said second position to permit said needle cannula (40,206) to be trapped in said shield by said cannula finger lock, and whereby said substantially rigid base end (222) of said cannula finger lock prevents re-exposure of said needle cannula after said shield is in said second position,
**characterized in that**
said at least one cannula finger lock (220) comprises at least first and second cannula finger locks,
a web (226) extends between portions of said first and second cannula finger locks (220) substantially adjacent the respective base ends (222) thereof for making said cannula finger locks (220) substantially rigid at locations in proximity to said base ends (222) thereof, and
portions of said cannula finger locks (220) adjacent the free ends (224) thereof are substantially free of said webs (226).

9. The safety needle assembly of claim 8, wherein said portions of said cannula finger lock (220) in proximity to said free end (224) are made more flexible than portions of said cannula finger lock (220) near said base end (222) by forming said cannula finger lock with reduced dimensions at locations in proximity to said free end (224) thereof.

10. The safety needle assembly of claim 9, wherein portions of said cannula finger lock (220) in proximity to said free end (224) thereof are resiliently deflectable in directions transverse to said needle cannula (40,206) when said shield (140a,140b) is rotated into said second position, said portions of said needle cannula in proximity to said free end (224) being of reduced dimension in directions extending substantially along a direction of deformation of said cannula finger lock (220).

11. The safety needle assembly of claim 9, wherein portions of said cannula finger lock (220) in proximity to said free end (224) thereof are resiliently deflectable in directions transverse to said needle cannula (40,206) when said shield (140a,140b) is rotated into said second position, said portions of said needle cannula in proximity to said free end (224) being of reduced dimension in directions substantially parallel to said needle cannula when said shield is in said second position.

12. The safety needle assembly of claim 8, wherein said cannula finger lock (220) is formed unitarily with said shield (140a,140b).

## Patentansprüche

1. Sicherheitsnadelanordnung mit einem Nadelansatz (60) mit einem proximalen und einem distalen Ende (64, 66) und einem sich zwischen den Enden erstreckenden Durchlass (62), einer Nadelkanüle (40), die an dem Durchlass (62) des Nadelansatzes (60) angebracht ist und ein spitzes distales Ende (44) aufweist, welches über das distale Ende (66) des Ansatzes (60) hinausragt, einem Schutz (140) mit einem proximalen und einem distalen Ende (144, 146), wobei das proximale Ende (144) des Schutzes (140) gelenkig an dem Ansatz (60) derart angebracht ist, dass es aus einer ersten Position, in welcher der Schutz von der Nadelkanüle beabstandet ist, in eine zweite Position drehbar ist, in welcher der Schutz die Nadelkanüle im Wesentlichen umgibt, wobei der Schutz (140) eine obere Wand (163) und einander gegenüberliegende erste und zweite Seitenwände (162) aufweist, die sich von der oberen Wand (163) aus erstrecken, wobei die Seitenwände (162) aufweisen: von der oberen Wand (163) entfernte Ränder (165), mindestens eine fingerbetätigbare Kanülenverriegelung (220) mit einem Basisende (222), das mit der ersten Seitenwand (162) einstückig ausgebildet ist, und einem freien Ende (224), das unter einem Winkel in Richtung der oberen Wand (163) ragt, wobei nahe dem Basisende (222) gelegene Bereiche der fingerbetätigbaren Kanülenverriegelung (220) im Wesentlichen starr sind und nahe dem freien Ende (224) gelegene Bereiche der fingerbetätigbaren Kanülenverriegelung (220) elastisch auslenkbar sind, wobei elastisch auslenkbare Bereiche der fingerbetätigbaren Kanülenverriegelung (220) nachgeben, wenn der Schutz (140) in die zweite Position gedreht wird, um das Fangen der Nadelkanüle (40) zwischen der fingerbetätigbaren Kanülenverriegelung (220) und der oberen Wand (163) zu ermöglichen, und wobei im Wesentlichen starre Bereiche der fingerbetätigbaren Kanülenverriegelung (220) das erneute Freilegen der Nadelkanüle (40) verhindern, nachdem sich der Schutz (140) in der zweiten Position befindet,
**dadurch gekennzeichnet, dass**
die mindestens eine fingerbetätigbare Kanülenverriegelung (220) mindestens eine erste und eine zweite fingerbetätigbare Kanülenverriegelung aufweist,
ein Steg (226) sich zwischen Bereichen der ersten und der zweiten fingerbetätigbaren Kanülenverriegelung (220) im Wesentlichen nahe den jeweiligen Basisenden (222) derselben erstreckt, um die fingerbetätigbaren Kanülenverriegelungen (220) an Stellen nahe den Basisenden (222) derselben im Wesentlichen starr zu machen, und
Bereiche der fingerbetätigbaren Kanülenverriegelungen (220) nahe den freien Enden (224) im Wesentlichen frei von den Stegen (226) sind.

2. Sicherheitsnadelanordnung nach Anspruch 1, bei welcher nahe dem freien Ende (224) gelegene Bereiche der fingerbetätigbaren Kanülenverriegelung (220) flexibler ausgebildet sind als nahe dem Basisende (222) gelegene Bereiche der fingerbetätigbaren Kanülenverriegelung (220), indem die fingerbetätigbare Kanülenverriegelung an Stellen nahe dem freien Ende (224) derselben mit verringerten Abmessungen ausgebildet ist.

3. Sicherheitsnadelanordnung nach Anspruch 2, bei welcher nahe dem freien Ende (224) gelegene Bereiche der fingerbetätigbaren Kanülenverriegelung (220) in quer zur Nadelkanüle (40) verlaufenden Richtungen elastisch auslenkbar sind, wenn der Schutz (140) in die zweite Position gedreht wird, wobei die nahe dem freien Ende (224) gelegenen Bereiche der Nadelkanüle (40) in Richtungen, die im Wesentlichen entlang der Verformungsrichtung der nahe dem freien Ende (224) gelegenen Bereiche der fingerbetätigbaren Kanülenverriegelung (220) verlaufen, eine geringere Abmessung aufweisen.

4. Sicherheitsnadelanordnung nach Anspruch 2, bei welcher nahe dem freien Ende (224) gelegenen Bereiche der fingerbetätigbaren Kanülenverriegelung (220) in quer zur Nadelkanüle (40) verlaufenden Richtungen elastisch auslenkbar sind, wenn der Schutz (140) in die zweite Position gedreht wird, wobei die nahe dem freien Ende (224) gelegenen Bereiche der Nadelkanüle (40) bei in der zweiten Position befindlichem Schutz (140) in Richtungen, die im Wesentlichen parallel zu der Nadelkanüle (40) verlaufen, eine geringere Abmessung aufweisen.

5. Sicherheitsnadelanordnung nach Anspruch 1, bei welcher die fingerbetätigbare Kanülenverriegelung (220) einstückig mit dem Schutz (140) ausgebildet ist.

6. Sicherheitsnadelanordnung nach Anspruch 1, bei welcher die Nadelkanüle (40) ein proximales Ende (42) aufweist, das proximal über die Nabe (60) hinaus ragt.

7. Sicherheitsnadelanordnung nach Anspruch 1, bei welcher der Ansatz (60) ferner mindestens eine daran ausgebildete Klinke (118) aufweist, und wobei der Schutz (140) einen daran ausgebildeten Ansatz (194) aufweist, der ausgebildet ist, um an der Klinke an dem Ansatz (60) anzugreifen, wenn der Schutz (140) sich in der zweiten Position befindet, wobei die Klinke (118) und der Ansatz (194) zum Bewirken einer hörbaren und fühlbaren Anzeige des Erreichens der zweiten Position des Schutzes (140) vorgesehen sind.

8. Sicherheitsnadelanordnung mit einer medizinischen Vorrichtung (200, 300), einer an der medizinischen Vorrichtung (200, 300) angebrachten Nadelkanüle (40, 206) mit einem spitzen distalen Ende (44), das über die medizinische Vorrichtung hinaus ragt, einem Schutz (140a, 140b) mit einem länglichen Schlitz (160), wobei der Schlitz (160) zum Aufnehmen zumindest des spitzen distalen Endes (44) der Nadelkanüle (40, 206) bemessen ist, wobei der Schutz (140a, 140b) gelenkig nahe der medizinischen Vorrichtung (200, 300) derart angebracht ist, dass er aus einer ersten Position, in welcher die Nadelkanüle freiliegt, in eine zweite Position drehbar ist, in welcher zumindest das spitze distale Ende (44) der Nadelkanüle sich in dem Schlitz (160) des Schutzes (140) befindet, mindestens eine fingerbetätigbare Kanülenverriegelung (220) mit einem im Wesentlichen starren Basisende (222), das von dem Schutz (140a, 140b) absteht und teilweise in den Schlitz (160) hinein ragt, wobei die fingerbetätigbare Kanülenverriegelung (220) ferner ein elastisch auslenkbares freies Ende (224) aufweist, wobei das elastisch auslenkbare freie Ende (224) der fingerbetätigbaren Kanülenverriegelung (220) nachgibt, wenn der Schutz (140a, 140b) in die zweite Position gedreht wird, um das Fangen der Nadelkanüle (40, 206) in dem Schutz durch die fingerbetätigbare Kanülenverriegelung zu ermöglichen, und wobei das im Wesentlichen starre Basisende (222) der fingerbetätigbaren Kanülenverriegelung ein erneutes Freilegen der Nadelkanüle verhindert, nachdem sich der Schutz in der zweiten Position befindet,
**dadurch gekennzeichnet, dass**
die mindestens eine fingerbetätigbare Kanülenverriegelung (220) mindestens eine erste und eine zweite fingerbetätigbare Kanülenverriegelung aufweist,
ein Steg (226) sich zwischen Bereichen der ersten und der zweiten fingerbetätigbaren Kanülenverriegelung (220) im Wesentlichen nahe den jeweiligen Basisenden (222) derselben erstreckt, um die fingerbetätigbaren Kanülenverriegelungen (220) an Stellen nahe den Basisenden (222) derselben im Wesentlichen starr zu machen, und
Bereiche der fingerbetätigbaren Kanülenverriegelungen (220) nahe den freien Enden (224) im Wesentlichen frei von den Stegen (226) sind.

9. Sicherheitsnadelanordnung nach Anspruch 8, bei welcher nahe dem freien Ende (224) gelegene Bereiche der fingerbetätigbaren Kanülenverriegelung (220) flexibler ausgebildet sind als nahe dem Basisende (222) gelegene Bereiche der fingerbetätigbaren Kanülenverriegelung (220), indem die fingerbetätigbare Kanülenverriegelung an Stellen nahe dem freien Ende (224) derselben mit verringerten Abmessungen ausgebildet ist.

10. Sicherheitsnadelanordnung nach Anspruch 9, bei welcher nahe dem freien Ende (224) gelegene Bereiche der fingerbetätigbaren Kanülenverriegelung (220) in quer zur Nadelkanüle (40, 206) verlaufenden Richtungen elastisch auslenkbar sind, wenn der Schutz (140a, 140b) in die zweite Position gedreht wird, wobei die nahe dem freien Ende (224) gelegenen Bereiche der Nadelkanüle in Richtungen, die im Wesentlichen entlang der Verformungsrichtung der fingerbetätigbaren Kanülenverriegelung (220) verlaufen, eine geringere Abmessung aufweisen.

11. Sicherheitsnadelanordnung nach Anspruch 9, bei welcher nahe dem freien Ende (224) gelegene Bereiche der fingerbetätigbaren Kanülenverriegelung (220) in quer zur Nadelkanüle (40, 206) verlaufenden Richtungen elastisch auslenkbar sind, wenn der Schutz (140a, 140b) in die zweite Position gedreht wird, wobei die nahe dem freien Ende (224) gelegenen Bereiche der Nadelkanüle bei in der zweiten Position befindlichem Schutz in Richtungen, die im Wesentlichen parallel zu der Nadelkanüle verlaufen, eine geringere Abmessung aufweisen.

12. Sicherheitsnadelanordnung nach Anspruch 8, bei welcher die fingerbetätigbare Kanülenverriegelung (220) einstückig mit dem Schutz (140a, 140b) ausgebildet ist.

## Revendications

1. Ensemble aiguilles sécurisé comprenant un embout d'aiguille (60) avec des extrémités proximales et distales (64, 66) et un passage (62) s'étendant entre lesdites extrémités, une canule d'aiguille (40) montée audit passage (62) dudit embout d'aiguille (60) et comprenant une extrémité distale pointue (44) saillant au-delà de l'extrémité distale (66) dudit embout (60), un protecteur (140) avec des extrémités proximales et distales (144, 146), ladite extrémité proximale (144) dudit protecteur (140) étant montée de manière articulée audit embout (60) pour permettre la rotation à partir d'une première position, dans laquelle ledit protecteur est écarté de ladite canule d'aiguille, vers une deuxième position, dans laquelle ledit protecteur sensiblement entoure ladite canule d'aiguille, ledit protecteur (140) comprenant une paroi de dessus (163) et des premières et deuxièmes parois latérales (162) opposées s'étendant de ladite paroi de dessus (163), lesdites parois latérales (162) comprenant des bords (165) loin de ladite paroi de dessus (163), au moins un moyen de verrouillage digital de canule (220) comprenant une extrémité de base (222) formée en une pièce avec la première paroi latérale (162) et une extrémité libre (224) saillant angulairement vers ladite paroi de dessus (163), des parties dudit moyen de verrouillage digital de canule (220) à proximité de ladite extrémité de base (222) étant sensiblement rigides, et des parties dudit moyen de verrouillage digital de canule (220) à proximité de ladite extrémité libre (224) étant aptes à être déviées élastiquement, lesdites parties dudit moyen de verrouillage digital de canule (220), aptes à être déviées élastiquement, fléchissant lorsque ledit protecteur (140) est tourné dans la deuxième position pour permettre d'attraper ladite canule d'aiguille (40) entre ledit moyen de verrouillage digital de canule (220) et ladite paroi en dessus (163), et lesdites parties dudit moyen de verrouillage digital de canule (220) sensiblement rigides empêchant ladite canule d'aiguille (40) d'être exposée de nouveau après ledit protecteur (140) est dans la deuxième position,
**caractérisé en ce que**
ledit au moins un moyen de verrouillage digital de canule (220) comprend au moins un premier et un deuxième moyen de verrouillage digital de canule,
une âme (226) s'étend entre des parties du premier et du deuxième moyen de verrouillage digital de canule (220) sensiblement près de leurs extrémités de base (222) respectives pour rendre lesdits moyens de verrouillage digital de canule (220) sensiblement rigides à des positions près de leurs extrémités de base (222), et
des parties desdits moyens de verrouillage digital de canule (220) près de leurs extrémités libres (224) sont sensiblement libres desdites âmes (226).

2. Ensemble aiguille sécurisé selon la revendication 1, dans lequel des parties dudit moyen de verrouillage digital de canule (220) à proximité de ladite extrémité libre (224) sont rendues plus flexibles que des parties dudit moyen de verrouillage digital de canule (220) à proximité de ladite extrémité de base (222) en formant ledit moyen de verrouillage digital de canule avec des dimensions réduites à positions près de son extrémité libre (224).

3. Ensemble aiguille sécurisé selon la revendication 2, dans lequel des parties dudit moyen de verrouillage digital de canule (220) à proximité de ladite extrémité libre (224) sont aptes à être déviées élastiquement dans des directions transversales à ladite canule d'aiguille (40) lorsque ledit protecteur (140) est tourné dans la deuxième position, lesdites parties de ladite canule d'aiguille (40) à proximité de ladite extrémité libre (224) ayant une dimension réduite dans des directions sensiblement le long d'une direction de déformation dudit moyen de verrouillage digital de canule (220).

4. Ensemble aiguille sécurisé selon la revendication 2, dans lequel des parties dudit moyen de verrouillage digital de canule (220) à proximité de ladite extrémité libre (224) sont aptes à être déviées élastiquement dans des directions transversales à ladite canule d'aiguille (40) lorsque ledit protecteur (140) est tourné dans la deuxième position, lesdites parties de ladite canule d'aiguille (40) à proximité de ladite extrémité libre (224) ayant une dimension réduite dans des directions sensiblement parallèles à ladite canule d'aiguille (40) quand ledit protecteur (140) est dans sa deuxième position.

5. Ensemble aiguille sécurisé selon la revendication 1, dans lequel ledit moyen de verrouillage digital de canule (220) est formé d'un seul tenant avec ledit protecteur (140).

6. Ensemble aiguille sécurisé selon la revendication 1, dans lequel ladite canule d'aiguille (40) a une extrémité proximale (42) saillant au-delà dudit embout (60) dans la direction proximale.

7. Ensemble aiguille sécurisé selon la revendication 1, dans lequel ledit embout (60) comprend en outre au moins un cran d'arrêt (118) formé sur celui, et dans lequel ledit protecteur (140) comprend une saillie (194) formée sur celui et configurée pour engager ledit cran d'arrêt sur ledit embout (60) quand ledit protecteur (140) est dans ladite deuxième position, ledit cran d'arrêt (118) et ladite saillie (194) étant configurés pour fournir une indication audible et tactile du fait que ledit protecteur (140) a atteint ladite deuxième position.

8. Ensemble aiguille sécurisé comprenant un dispositif médical (200, 300), une canule d'aiguille (40, 206) montée au dispositif médical (200, 300) et comprenant une extrémité distale pointue (44) saillant au-delà dudit dispositif médical, un protecteur (140a, 140b) avec une fente longitudinale (160), ladite fente (160) étant dimensionnée pour recevoir au moins ladite extrémité distale pointue (40) de ladite canule d'aiguille (40, 206), ledit protecteur (140a, 140b) étant monté de manière articulée à proximité dudit dispositif médical (200, 300) pour permettre la rotation à partir d'une première position, dans laquelle ladite canule d'aiguille est exposée, vers une deuxième position, dans laquelle au moins ladite extrémité distale pointue (44) de ladite canule d'aiguille est dans ladite fente (160) dudit protecteur (140), au moins moyen de verrouillage digital de canule (220) comprenant une extrémité de base (222) sensiblement rigide saillant dudit protecteur (140a, 140b) et partiellement dans ladite fente (160), ledit moyen de verrouillage digital de canule (220) comprenant en outre une extrémité libre (224) apte à être déviée élastiquement, ladite extrémité libre (224) dudit moyen de verrouillage digital de canule (220) fléchissant lorsque ledit protecteur (140a, 140b) est tourné dans la deuxième position pour permettre d'attraper ladite canule d'aiguille (40, 206) dans ledit protecteur à l'aide dudit moyen de verrouillage digital de canule, et ladite extrémité de base (222) dudit moyen de verrouillage digital de canule, sensiblement rigide, empêchant ladite canule d'aiguille d'être exposée de nouveau après ledit protecteur est dans la deuxième position,
**caractérisé en ce que**
ledit au moins un moyen de verrouillage digital de canule (220) comprend au moins un premier et un deuxième moyen de verrouillage digital de canule,
une âme (226) s'étend entre des parties du premier et du deuxième moyen de verrouillage digital de canule (220) sensiblement près de leurs extrémités de base (222) respectives pour rendre lesdits moyens de verrouillage digital de canule (220) sensiblement rigides à des positions près de leurs extrémités de base (222), et
des parties desdits moyens de verrouillage digital de canule (220) près de leurs extrémités libres (224) sont sensiblement libres desdites âmes (226).

9. Ensemble aiguille sécurisé selon la revendication 8, dans lequel les parties dudit moyen de verrouillage digital de canule (220) à proximité de ladite extrémité libre (224) sont rendues plus flexibles que les parties dudit moyen de verrouillage digital de canule (220) à proximité de ladite extrémité de base (222) en formant ledit moyen de verrouillage digital de canule avec des dimensions réduites à positions près de son extrémité libre (224).

10. Ensemble aiguille sécurisé selon la revendication 9, dans lequel des parties dudit moyen de verrouillage digital de canule (220) à proximité de ladite extrémité libre (224) sont aptes à être déviées élastiquement dans des directions transversales à ladite canule d'aiguille (40, 206) lorsque ledit protecteur (140a, 140b) est tourné dans la deuxième position, lesdites parties de ladite canule d'aiguille à proximité de ladite extrémité libre (224) ayant une dimension réduite dans des directions sensiblement le long d'une direction de déformation dudit moyen de verrouillage digital de canule (220).

11. Ensemble aiguille sécurisé selon la revendication 9, dans lequel des parties dudit moyen de verrouillage digital de canule (220) à proximité de ladite extrémité libre (224) sont aptes à être déviées élastiquement dans des directions transversales à ladite canule d'aiguille (40, 206) lorsque ledit protecteur (140a, 140b) est tourné dans la deuxième position, lesdites parties de ladite canule d'aiguille à proximité de ladite extrémité libre (224) ayant une dimension réduite dans des directions sensiblement parallèles à ladite canule d'aiguille quand ledit protecteur est dans sa deuxième position.

12. Ensemble aiguille sécurisé selon la revendication 8, dans lequel ledit - moyen de verrouillage digital de canule (220) est formé d'un seul tenant avec ledit protecteur (140a, 140b).
